# EUROPÄISCHE PATENTANMELDUNG

(11) **EP 3 023 902 A1**
(43) Veröffentlichungstag der Anmeldung: **25.05.2016**
(21) Anmeldenummer: 14194513.9
(22) Anmeldetag: 24.11.2014
(51) Int. Cl.: G06F 21/62, G06F 19/00

(54) **Verfahren zum Anonymisieren von medizinischen Datensätzen**

(71) Anmelder: Siemens Aktiengesellschaft, 80333 München (DE)
(72) Erfinder: Goßler, Thomas, 91056 Erlangen (DE); Hewett, Andrew John, 91058 Erlangen (DE); Schottlander, David, Kidlington, Oxfordshire, OX5 1EN (GB); Ukis, Vladyslav, 90489 Nürnberg (DE)

(57) **Zusammenfassung**

Die vorliegende Erfindung betrifft ein Verfahren zum Anonymisieren von medizinischen Datensätzen, mit den Schritten eines Anonymisierens (S101) eines Patientendatensatzes durch Abtrennen der Patientenidentifikationsdaten aus einem Patientendatensatz, der Patientenidentifikationsdaten und Patientendaten umfasst, auf Basis einer Anonymisierungsregel; eines Einbettens (S102) der verwendeten Anonymisierungsregel in den anonymisierten Patientendatensatz; und eines Übertragens (S103) des anonymisierten Patientendatensatzes mit der eingebetteten Anonymisierungsregel an einen Datenspeicher (111).

## Beschreibung

Die vorliegende Erfindung betrifft ein Verfahren zum Anonymisieren von medizinischen Datensätzen.

Die Druckschrift US 2011/0119088 A1 betrifft ein Verfahren zum Austauschen von Gesundheitsinformation über ein Gesundheitsaustauschsystem. Das Gesundheitsaustauschsystem dient dazu, medizinische Information zwischen unterschiedlichen Einheiten auszutauschen, die in unterschiedlichen Formaten gespeichert ist. Das Austauschsystem stellt einen Kanal für den Fluss von Information und Patientendatensätzen über unterschiedliche Gesundheitseinrichtungen bereit, die Daten in unterschiedlichen Formaten speichern.

Im Gesundheitswesen existieren strenge Regelungen, dass geschützte Gesundheitsinformation (PHI - Protected Health Information) nicht außerhalb eines Landes oder gar außerhalb eines Krankenhauses gespeichert werden darf, in dem die Daten erzeugt worden sind. Dieser Grundsatz schließt die Möglichkeit aus, externe Datencenter zum Speichern und Verarbeiten dieser Daten zu verwenden. Öffentliches Cloud-Computing ist ein moderner Ansatz zum effizienten Betreiben von Software in einer skalierbaren und elastischen Weise, so dass die Betriebskosten direkt mit der Speichermenge und dem Berechnungsressourcen in Beziehung stehen. Diese Betriebsweise weist Vorteile für medizinische Einrichtungen auf. Aufgrund strenger Regulierungen kann dieser Ansatz nicht für personalisierte Patientendaten verwendet werden.

Krankenhäuser müssen daher aufwendige Hardware kaufen, Personal zum Betrieb einstellen und die Hardware alle paar Jahre erneuern. Dies ist technisch ineffizient. Daneben werden Datensilos erzeugt, die große Mengen an Daten umfassen, die nur schwer mit anderen Institutionen zu teilen sind. Zum Teilen der Daten unter den Einrichtungen werden üblicherweise CD-ROMs verwendet, die in mehreren Tagen mit der Post verschickt werden. Bei anonymisierten Patientendatensätzen kann zudem technisch nicht überprüft werden, ob diese korrekt anonymisiert worden sind.

Es ist die Aufgabe der vorliegenden Erfindung, medizinische Datensätze auf einfache Weise hinsichtlich ihrer Anonymität mit einem geringen Einsatz von technischen Ressourcen zu überprüfen.

Gemäß einem ersten Aspekt wird diese Aufgabe durch ein Verfahren zum Anonymisieren von medizinischen Datensätzen gelöst, mit den Schritten eines Anonymisierens eines Patientendatensatzes durch Abtrennen der Patientenidentifikationsdaten aus einem Patientendatensatz, der die Patientenidentifikationsdaten und Patientendaten umfasst, auf Basis einer Anonymisierungsregel; eines Einbettens der verwendeten Anonymisierungsregel in den anonymisierten Patientendatensatz; und eines Übertragens des anonymisierten Patientendatensatzes mit der eingebetteten Anonymisierungsregel an einen Datenspeicher. Dadurch wird beispielsweise der technische Vorteil erreicht, dass andere Einheiten den Patientendatensatz lokal und effizient anhand der Anonymisierungsregel überprüfen können. Die anonymisierten Patientendatensätze von unterschiedlichen medizinischen Einrichtungen können in einem externen Datenspeicher gespeichert und verarbeitet werden.

In einer vorteilhaften Ausführungsform des Verfahrens wird die verwendete Anonymisierungsregel an den anonymisierten Patientendatensatz angehängt. Dadurch wird beispielsweise der technische Vorteil erreicht, dass die Anonymisierungsregel auf einfache Weise in den anonymen Patientendatensatz eingebettet werden kann.

In einer weiteren vorteilhaften Ausführungsform des Verfahrens überprüft der Datenspeicher eine Anonymisierung des übertragenen Patientendatensatzes anhand der Anonymisierungsregel. Dadurch wird beispielsweise der technische Vorteil erreicht, dass die Anonymität der Patientendatensätze redundant von zwei unterschiedlichen Einheiten überprüft wird.

In einer weiteren vorteilhaften Ausführungsform des Verfahrens wendet der Datenspeicher eine Schrifterkennung auf Bilddaten des anonymisierten Patientendatensatz an, um die Anonymisierungsregel zu überprüfen. Dadurch wird beispielsweise der technische Vorteil erreicht, dass Schriftinformation aus Bildern berücksichtigt wird, die Patientenidentifikationsdaten umfassen kann.

In einer weiteren vorteilhaften Ausführungsform des Verfahrens versendet der Datenspeicher bei einem negativen Ergebnis der Überprüfung eine Nachricht. Dadurch wird beispielsweise der technische Vorteil erreicht, dass der Empfänger der Nachricht über die fehlgeschlagene Überprüfung informiert wird.

In einer weiteren vorteilhaften Ausführungsform des Verfahrens verwirft der Datenspeicher bei einem negativen Ergebnis der Überprüfung den anonymisierten Patientendatensatz. Dadurch wird beispielsweise der technische Vorteil erreicht, dass nur geprüfte anonymisierte Patientendatensätze in dem Datenspeicher gespeichert werden.

In einer weiteren vorteilhaften Ausführungsform des Verfahrens speichert der Datenspeicher bei einem positiven Ergebnis der Überprüfung den anonymisierten Patientendatensatz. Dadurch wird beispielsweise ebenfalls der technische Vorteil erreicht, dass nur geprüfte anonymisierte Patientendatensätze in dem Datenspeicher gespeichert werden.

In einer weiteren vorteilhaften Ausführungsform des Verfahrens wird der anonymisierte Patientendatensatz in einem Dateiordner mit einem eindeutigen Bezeichner gespeichert. Dadurch wird beispielsweise der technische Vorteil erreicht, dass auf den Patientendatensatz auf einfache Weise zugegriffen werden kann.

In einer weiteren vorteilhaften Ausführungsform des Verfahrens wird die Anonymisierungsregel bei einem Abrufen des anonymisierten Patientendatensatzes überprüft. Dadurch wird beispielsweise der technische Vorteil erreicht, dass ein Abruf von nicht-anonymisierten Daten verhindert wird.

In einer weiteren vorteilhaften Ausführungsform des Verfahrens wird bei einem negativen Ergebnis der Überprüfung ein Abruf des anonymisierten Patientendatensatzes verhindert. Dadurch wird beispielsweise ebenfalls der technische Vorteil erreicht, dass ein Abruf von nicht-anonymisierten Daten verhindert wird.

In einer weiteren vorteilhaften Ausführungsform des Verfahrens ist der Patientendatensatz oder der anonymisierte Patientendatensatz eine DICOM-Datei. Dadurch wird beispielsweise der technische Vorteil erreicht, dass ein besonders geeignetes Format zur Speicherung und zum Austausch von Informationen im medizinischen Bilddatenmanagement verwendet wird.

In einer weiteren vorteilhaften Ausführungsform des Verfahrens wird ein verschlüsselter Patientenidentifikationsdatensatz auf Basis der abgetrennten Patientenidentifikationsdaten erzeugt. Dadurch wird beispielsweise der technische Vorteil erreicht, dass die Patientenidentifikationsdaten weiterhin zum Personalisieren des Patientendatensatzes verwendet werden kann.

In einer weiteren vorteilhaften Ausführungsform des Verfahrens wird der verschlüsselte Patientenidentifikationsdatensatz zusammen mit dem anonymisierten Patientendatensatz übertragen. Dadurch wird beispielsweise der technische Vorteil erreicht, dass die anonymisierten Datensätze anhand der verschlüsselten Patienteninformationsdatensätze erneut personalisiert werden können.

In einer weiteren vorteilhaften Ausführungsform des Verfahrens ist der Patientenidentifikationsdatensatz eine JavaScript-Object-Notation-Datei. Dadurch wird beispielsweise der technische Vorteil erreicht, dass ein kompaktes Datenformat in einer einfach lesbaren Textform zum Zweck des Datenaustauschs zwischen Anwendungen verwendet wird.

Gemäß einem zweiten Aspekt wird die Aufgabe durch ein Computersystem zum Anonymisieren von medizinischen Datensätzen gelöst, mit einer Anonymisierungseinrichtung zum Anonymisieren eines Patientendatensatzes durch Abtrennen der Patientenidentifikationsdaten aus einem Patientendatensatz, der Patientenidentifikationsdaten und Patientendaten umfasst, auf Basis einer Anonymisierungsregel; einer Einbettungseinrichtung zum Einbetten der verwendeten Anonymisierungsregel in den anonymisierten Patientendatensatz; und einer Hochladeeinrichtung zum Übertragen des anonymisierten Patientendatensatzes mit der eingebetteten Anonymisierungsregel an einen Datenspeicher. Dadurch werden die gleichen technischen Vorteile wie durch das Verfahren nach dem ersten Aspekt gelöst.

Ausführungsbeispiele der Erfindung sind in den Zeichnungen dargestellt und werden im Folgenden näher beschrieben.

Es zeigen:
- Fig. 1: ein Blockdiagramm eines Verfahrens zum Anonymisieren von Datensätzen;
- Fig. 2: ein Datenflussdiagramm; und
- Fig. 3: ein Computersystem zum Anonymisieren von Patientendatensätzen.

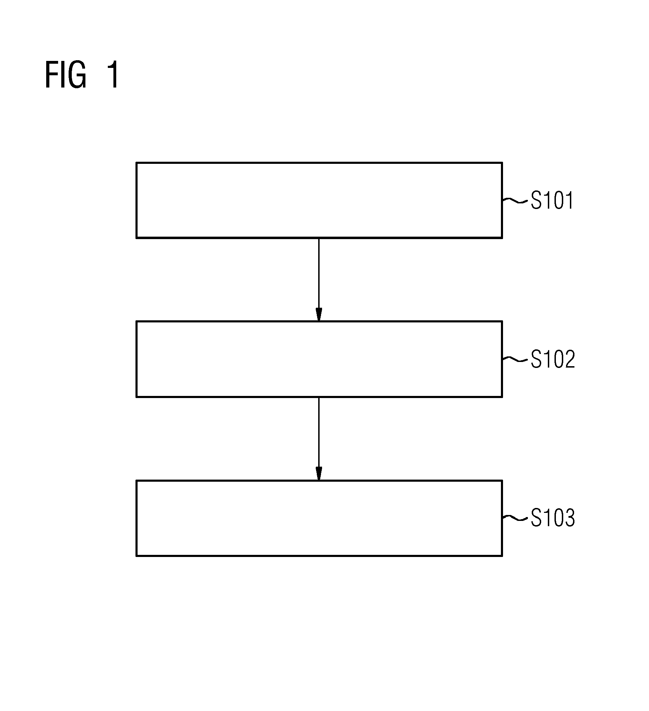

Fig. 1 zeigt ein Blockdiagramm eines Verfahrens zum Anonymisieren von Datensätzen. In einem ersten Schritt S101 wird ein neuer Patientendatensatz durch Abtrennen der Patientenidentifikationsdaten aus einem ursprünglichen Patientendatensatz auf Basis einer vorgegebenen Anonymisierungsregel erzeugt.

Der ursprüngliche Patientendatensatz umfasst beispielsweise die Patientenidentifikationsdaten und Patientenuntersuchungsdaten als weitere Patientendaten.

Die verwendete Anonymisierungsregel wird anschließend in Schritt S102 in den anonymisierten Patientendatensatz eingebettet. Danach wird in Schritt S103 der anonymisierte Patientendatensatz mit der eingebetteten Anonymisierungsregel an einen externen Datenspeicher übertragen. Der externe Datenspeicher ist beispielsweise eine Cloud oder ein Rechenzentrum im Internet.

Dadurch werden die sensitiven Patientenidentifikationsdaten von nicht-sensitiven Patientendaten getrennt. Die anonymisierten Patientendatensätze werden anschließend gesichert, so dass ein zentrales Speichern und effizientes Verarbeiten der Patientendatensätze in einer öffentlichen Cloud-Umgebung ermöglicht wird.

Die Patientenidentifikationsdaten können verschlüsselt werden, so dass lediglich der Eigentümer der Patientenidentifikationsdaten diese entschlüsseln und betrachten kann (End-zu-End-Verschlüsselung).

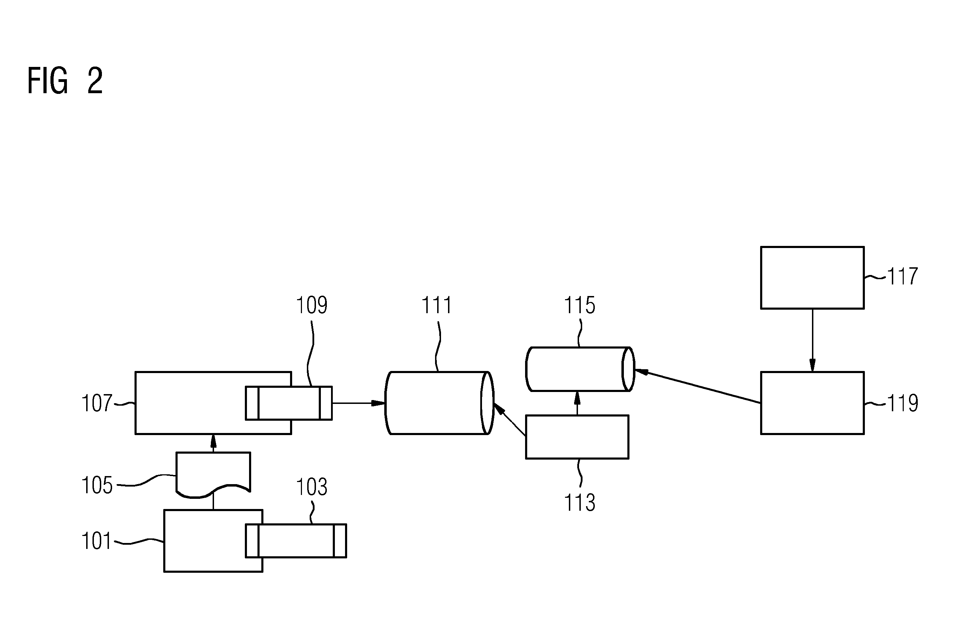

Fig. 2 zeigt einen gesamten Datenfluss. In dem Krankenhaus als lokale Einrichtung läuft eine Hochladeeinrichtung 101 auf einem lokalen Rechner. Die Hochladeeinrichtung 101 empfängt die Patientendatensätze 105 aus den lokalen Datenbanken, wie beispielsweise DICOM-Datensätze (DICOM - Digital Imaging and Communications in Medicine) aus einem PACS-System (PACS - Picture Archiving and Communication System) und lädt die Patientendatensätze 105 nach einer Anonymisierung an die Empfängereinrichtung 107 der Cloud hoch.

Vorher werden die Daten an eine Anonymisierungseinrichtung 103 als Subsystem übergeben, die die sensitiven Patientenidentifikationsdaten anhand vorgegebener Anonymisierungsregeln identifiziert, aus dem Patientendatensatz abtrennt und den ursprünglichen Patientendatensatz durch Entfernen der Patientenidentifikationsdaten anonymisiert. Die Patientenidentifikationsdaten werden als Patientenidentifikationsdatensatz mit einem Schlüssel verschlüsselt, der für die medizinische Einrichtung spezifisch ist.

Der nicht verschlüsselte Teil des Patientendatensatzes wird gemäß medizinischen Anonymisierungsstandards verarbeitet, so dass die gesamte sensitive Patientenidentifikationsdaten aus diesem entfernt ist. Die ursprünglichen Patientendatensätze können in unterschiedlicher Weise anhand unterschiedlicher vorgegebener Anonymisierungsregeln aus Sicherheitsprofilen oder Datenvertraulichkeitseinstufungen anonymisiert werden. Da die Anonymisierung auf unterschiedliche Weise durchgeführt werden kann, liegen im Allgemeinen keine gleichmäßig anonymisierten Patientendatensätze in der Cloud vor. Daher unterscheiden sich die Patientendatensätze hinsichtlich ihrer Anonymisierung.

Eine Datenverarbeitung in der Cloud und dem Client sollte daher die Art und Weise berücksichtigen, in der die Patientendatensätze anonymisiert worden sind, um Interpretationsfehler bei einer Verarbeitung zu vermeiden. Dies wird durch Einbetten oder Anhängen eines Satzes von Anonymisierungsregeln in den anonymisierten Patientendatensatz erreicht, die als ein privater Metadatenwert angewendet werden. Die privaten Metadaten als Anonymisierungsregeln können dann in der Cloud oder dem Client während einer Datenverarbeitung evaluiert werden.

Ein Beispiel ist die Anonymisierung eines Geburtsdatums des Patienten. Dieser Wert wird in jedem Patientendatensatz in einem vorgegebenen Format gespeichert. Anhand der Anonymisierungsregel kann das Geburtsdatum durch ein Pseudogeburtsdatum in dem anonymisierten Patientendatensatz ersetzt werden. Um bei einer späteren Verarbeitung der Patientendatensätze eine Fehlinterpretation zu verhindern, liest die Anwendung die eingebetteten Metadaten ein, die die verwendeten Anonymisierungsregeln spezifizieren.

Die sensitiven Patientenidentifikationsdaten werden vor einer Verschlüsselung als JSON-Datensatz formatiert, wenn diese von dem ursprünglichen Patientendatensatz, beispielsweise ein DICOM-Datensatz, getrennt werden. Dadurch kann später jede Art von Client, wie beispielsweise reine Web-Clients, effizient mit diesen Patientenidentifikationsdaten arbeiten. Insbesondere ist es hierzu nicht erforderlich, dass DICOM-Verarbeitungsbibliotheken auf der Clientseite verwendet werden. Falls das DICOM-Format beibehalten wird, geschieht dies, da das DICOM-Format aufgrund einer End-zu-End-Verschlüsselung auf der Clientseite nach einer Entschlüsselung behandelt wird.

Anschließend werden die verschlüsselten und die unverschlüsselten Daten über einen sicheren TLS/SSL-Kommunikationskanal in die Cloud hochgeladen. Die Empfängereinrichtung 107 validiert erneut mit der Validierungseinrichtung 109 das Nicht-Vorliegen von sensitiver Patienteninformation in den empfangenen unverschlüsselten Patientendatensätzen anhand der eingebetteten Anonymisierungsregel.

Dies kann eine optische Buchstabenerkennung (OCR - Optical Character Recognition) einschließen, so dass sogar sensitive Patienteninformation erfasst werden kann, die in medizinischen Bilddateien eingebettet ist. Falls eine Überprüfung oder Validierung fehlschlägt, werden die Daten sofort verworfen und gelöscht. Die Hochladeeinrichtung 101 wird über den fehlgeschlagenen Hochladevorgang benachrichtigt.

Falls die Überprüfung oder Validierung erfolgreich ist, werden die Daten unverändert in einem hochskalierbaren Datenspeicher 111 gespeichert. Die Struktur in diesem Datenspeicher 111 folgt einer einfachen und skalierbaren Namenskonvention. Die gesamten medizinischen Daten werden in Dateiordner gelegt, die für die medizinischen Studien spezifisch sind. Jeder Dateiordner wird gemäß dem eindeutigen Identifikator der entsprechenden Studie benannt. Innerhalb eines derartigen Dateiordners werden die Patientendatensätze der Studie in Dateien gespeichert, die gemäß dem eindeutigen Identifikator des Patientendatensatzes benannt werden. Weitere Unterdateiordner werden nicht verwendet. Für jeden Datensatz existieren getrennte Dateien für den anonymisierten Patientendatensatz und den verschlüsselten, sensitiven Patientenidentifikationsdatensatz.

Dieser Ansatz ermöglicht, dass keine komplexen Datenbankstrukturen bereitgestellt werden müssen, um die Daten effizient abzurufen. Eine Studie umfasst eine begrenzte Anzahl von Datensätzen, so dass die Studie nicht überladen wird. Die Anzahl der Dateiordner für die Studien ist unbegrenzt und kann über die Zeit hinweg wachsen. Auf diese Weise ist das Konzept hochskalierbar und effizient.

Eine Struktur der Daten in dem Datenspeicher 111 ist beispielsweise wie folgt:

**<Root>**

| | |
|---|---|
| \|--- Study_<Studienidentifikator 1> | |
| \|--- <Datensatzidentifikator 1>.json | unverschlüsselte |
| \| | anonymisierte |
| | Metadaten |
| \|--- <Datensatzidentifikator 1>.phi.json | verschlüsselte |
| | sensitive |
| \| | Metadaten |
| \|--- <Datensatzidentifikator 1>.png | unverschlüsselte |
| \| | anonymisierte |
| | Bilddaten |
| \|--- <Datensatzidentifikator 1>.dicom | verschlüsselte ursprüngliche medizinischen Daten (Metadaten+Bilddaten) |

Die Empfängereinrichtung 107 sendet eine Benachrichtigung an die Extrahierungseinrichtung 113, nachdem die neuen Daten angekommen sind. Die Extrahierungseinrichtung 113 verarbeitet dann die neuen Daten in dem Datenspeicher 111. Das Ergebnis ist ein aktualisierter Suchindex in der Indexdatenbank 115, die es Web-Diensten erlaubt, Informationen flexibel und schnell aus dem Datenspeicher 111 abzurufen.

Eine Anwendung, die von Benutzern verwendet wird und auf dem Client 117 läuft, kann durch eine Webseite, eine native mobile Anwendung oder eine klassische Desktopanwendung gebildet sein. Eine Anwendungssoftware ruft die kleinen oder großen Datensätze über einen Web-Dienst 119 ab. Große Datensätze können auch aus dem Datenspeicher 111 direkt abgerufen werden. Um dies zu ermöglichen, beziehen sich die kleinen Datensätze, die von dem Web-Dienst 119 an den Client 117 übergeben werden, auf große Datensätze mittels einer URL (URL - Uniform Ressource Locator). Falls die Anwendung auf dem Client 117 später die referenzierten großen Datensätze laden möchte, kann diese die URL verwenden, um diese direkt aus dem Datenspeicher 111 abzurufen. Dieser Ansatz senkt den Berechnungsaufwand und ist skalierbar.

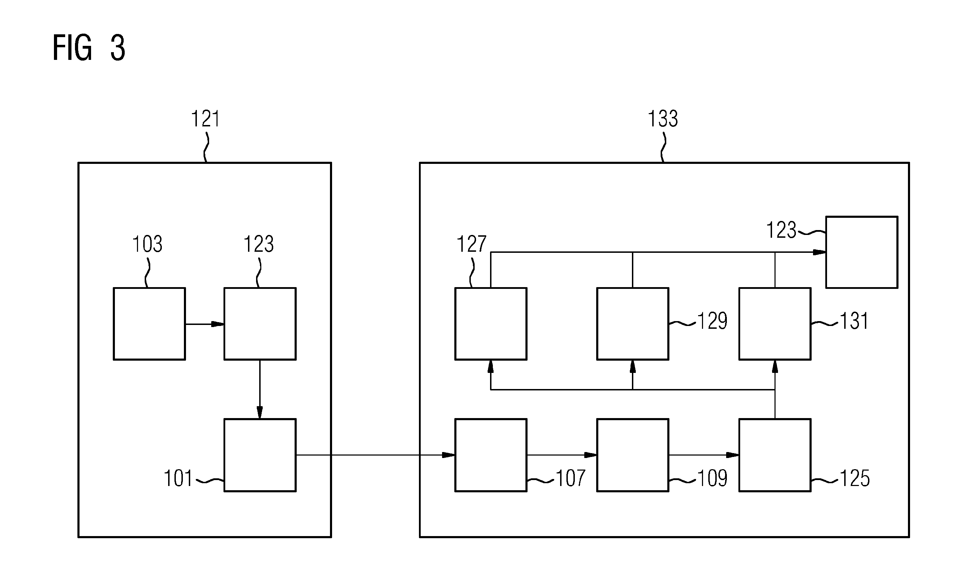

Fig. 3 zeigt ein Computersystem zum Anonymisieren von Patientendatensätzen. Die Anonymisierungseinrichtung 103 und die Hochladeeinrichtung 101 sind ein Teil einer Anwendung 121 eines Krankenhauses. Die Krankenhausanwendung umfasst zusätzlich eine Anonymisierungsregel-Einbettungseinrichtung 123, die für jeden Patientendatensatz die Art der Anonymisierung, die von der Anonymisierungseinrichtung 103 durchgeführt worden ist, in ein spezielles privates DICOM-Feld einsetzt.

Die Cloud 133 umfasst die Empfängereinrichtung 107, die Validierungseinrichtung 109 und eine Benachrichtigungseinrichtung 125, die eine Nachricht bei der Verfügbarkeit neuer Patientendatensätze sendet. Weiter umfasst die Cloud ein Dosierungsverwaltungs-Backend 127, ein Scannerverwendungs-Backend 129 und ein Bildverwendungs-Backend 131. Zudem umfasst die Cloud 133 ebenfalls eine Anonymisierungsregel-Einbettungseinrichtung 123. Die Anonymisierungseinrichtung 103, die Hochladeeinrichtung 101 und die Anonymisierungsregel-Einbettungseinrichtung 123 können durch ein ausführbares Programm oder eine elektrische Schaltung gebildet sein.

Für jeden anonymisierten Patientendatensatz wird aus dem privaten DICOM-Feld bestimmt, welche Art der Anonymisierung von der Anwendung durchgeführt worden ist. Ein Verarbeiten des anonymisierten Patientendatensatzes erfolgt auf Basis der zuvor bestimmten Anonymisierungsregel.

Das Verfahren ermöglicht ein Teilen von Patientendatensätzen zwischen unterschiedlichen medizinischen Einrichtungen und einzelnen Benutzern. Die Patientendatensätze können beispielsweise durch einen Scanner einer Computertomografie oder einer Magnetresonanztomografie oder ein Röntgen- oder Ultraschallverfahren erzeugt werden. Das Verfahren ermöglicht es anhand der eingebetteten Anonymisierungsregel mit wenigen Rechenschritten lokal zu überprüfen, ob die Patientendatensätze korrekt anonymisiert worden sind. Das Verfahren ermöglicht es zudem, dass Krankenhäuser in einer neuen Weise zusammenarbeiten.

Vorteilhaft ist dabei, dass eine Trennung von sensitiven Patientenidentifikationsdaten PHI von nicht-sensitiven Patientendaten erfolgt, so dass ein Speichern und Verarbeiten in einer Cloud-Umgebung ermöglicht wird. Die Daten können in ein verbreitetes Web-Dokumentenformat gebracht werden, so dass diese effizient von jedem Client verarbeitet werden können.

Durch das Anhängen von verwendeten Anonymisierungsregeln an die anonymisierten Patientendatensätze kann jede Verarbeitungsanwendung bestimmen, welche Teile der Patientendaten verarbeitet werden können. Zudem wird eine zweite Überprüfung der anonymisierten Patientendatensätze bei einem Übertragen der Daten in einen Datenspeicher ermöglicht. Der Datenspeicher kann den anonymisierten Patientendatensatz automatisch verwerfen, wenn bei der Überprüfung Probleme auftauchen. Eine einfache und effiziente Speicherstruktur und Identifikation erlaubt einen schnellen und skalierbaren Zugriff auf die Daten, obwohl diese End-zu-End-verschlüsselt werden. Die Verwendung eines skalierbaren Indizierungsmechanismus ermöglicht eine schnelle Suche und ein Browsen durch die Patientendaten. Zudem kann ein effizienter zweiseitiger Zugriff auf die Patientendaten basierend auf der Größe der Daten erzielt werden, beispielsweise in einem Web-Dienst oder einem direkten BLOB-Speicherzugriff.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

### Bezugszeichenliste

- 101: Hochladeeinrichtung
- 103: Anonymisierungseinrichtung
- 105: Patientendatensätze
- 107: Empfängereinrichtung
- 109: Validierungseinrichtung
- 111: Datenspeicher
- 113: Extrahierungseinrichtung
- 115: Indexdatenbank
- 117: Client
- 119: Web-Dienst
- 121: Anwendung
- 123: Anonymisierungsregel-Einbettungseinrichtung
- 125: Benachrichtigungseinrichtung
- 127: Dosierungsverwaltungs-Backend
- 129: Scannerverwendungs-Backend
- 131: Bildverwendungs-Backend
- 133: Cloud

## Patentansprüche

1. Verfahren zum Anonymisieren von medizinischen Datensätzen, mit den Schritten:
Anonymisieren (S101) eines Patientendatensatzes durch Abtrennen der Patientenidentifikationsdaten aus einem Patientendatensatz, der Patientenidentifikationsdaten und Patientendaten umfasst, auf Basis einer Anonymisierungsregel;
Einbetten (S102) der verwendeten Anonymisierungsregel in den anonymisierten Patientendatensatz; und
Übertragen (S103) des anonymisierten Patientendatensatzes mit der eingebetteten Anonymisierungsregel an einen Datenspeicher (111).

2. Verfahren nach Anspruch 1, wobei die verwendete Anonymisierungsregel an den anonymisierten Patientendatensatz angehängt wird.

3. Verfahren nach einem der vorangehenden Ansprüche, wobei der Datenspeicher eine Anonymisierung des übertragenen Patientendatensatzes anhand der Anonymisierungsregel überprüft.

4. Verfahren nach Anspruch 3, wobei der Datenspeicher eine Schrifterkennung auf Bilddaten des anonymisierten Patientendatensatz anwendet, um die Anonymisierungsregel zu überprüfen.

5. Verfahren nach Anspruch 3 oder 4, wobei der Datenspeicher bei einem negativen Ergebnis der Überprüfung eine Nachricht versendet.

6. Verfahren nach einem der Ansprüche 3 bis 5, wobei der Datenspeicher bei einem negativen Ergebnis der Überprüfung den anonymisierten Patientendatensatz verwirft.

7. Verfahren nach einem der Ansprüche 3 bis 6, wobei der Datenspeicher bei einem positiven Ergebnis der Überprüfung den anonymisierten Patientendatensatz speichert.

8. Verfahren nach einem der vorangehenden Ansprüche, wobei der anonymisierte Patientendatensatz in einem Dateiordner mit einem eindeutigen Bezeichner gespeichert wird.

9. Verfahren nach einem der vorangehenden Ansprüche, wobei die Anonymisierungsregel bei einem Abrufen des anonymisierten Patientendatensatzes überprüft wird.

10. Verfahren nach Anspruch 9, wobei bei einem negativen Ergebnis der Überprüfung ein Abruf des anonymisierten Patientendatensatzes verhindert wird.

11. Verfahren nach einem der vorangehenden Ansprüche, wobei der Patientendatensatz oder der anonymisierte Patientendatensatz eine DICOM-Datei ist.

12. Verfahren nach einem der vorangehenden Ansprüche, wobei ein verschlüsselter Patientenidentifikationsdatensatz auf Basis der abgetrennten Patientenidentifikationsdaten erzeugt wird.

13. Verfahren nach Anspruch 12, wobei der verschlüsselte Patientenidentifikationsdatensatz zusammen mit dem anonymisierten Patientendatensatz übertragen wird.

14. Verfahren nach einem der Ansprüche 12 oder 13, wobei der Patientenidentifikationsdatensatz eine JavaScript-Object-Notation-Datei ist.

15. Computersystem zum Anonymisieren von medizinischen Datensätzen, mit:
einer Anonymisierungseinrichtung (103) zum Anonymisieren eines Patientendatensatzes durch Abtrennen der Patientenidentifikationsdaten aus einem Patientendatensatz, der Patientenidentifikationsdaten und Patientendaten umfasst, auf Basis einer Anonymisierungsregel;
einer Einbettungseinrichtung (123) zum Einbetten der verwendeten Anonymisierungsregel in den anonymisierten Patientendatensatz; und
einer Hochladeeinrichtung (101) zum Übertragen des anonymisierten Patientendatensatzes mit der eingebetteten Anonymisierungsregel an einen Datenspeicher (111).
